# EUROPEAN PATENT SPECIFICATION

(11) **EP 1 119 537 B1**
(45) Date of publication and mention of the grant of the patent: **23.07.2003**
(21) Application number: 99954565.0
(22) Date of filing: 28.09.1999
(51) Int. Cl.: C07C 41/42, C07C 43/15

(54) **METHOD OF RECOVERY OF TRIMETHYLOLPROPANE TRIALLYL OR DIALLYL ETHER**
VERFAHREN ZUR RÜCKGEWINNUNG VON TRIMETHYLOLPROPAN-TRIALLYL- ODER -DIALLYL-ETHER
PROCEDE DE RECUPERATION D'ETHER TRIALLYLE OU DIALLYLE DE TRIMETHYLOL-PROPANE

(30) Priority: 30.09.1998 SE 9803315
(43) Date of publication of application: 01.08.2001
(73) Proprietor: PERSTORP AB, 284 80 Perstorp (SE)
(72) Inventor: Möllby, Nils, 113 32 Stockholm (SE); Rasmuson, Äke, 100 44 Stockholm (SE); Pajalic, Oleg S., 291 59 Kristianstad (SE)
(74) Representative: HOFFMANN - EITLE
(86) International application number: SE9901711
(87) International publication number: WO00018713

(56) References cited:
- GB-A- 1 473 024

## Description

The present invention refers to a method used in recovery of trimethylolpropane triallyl ether and/or trimethylolpropane diallyl ether from a mixture comprising said triallyl ether and said diallyl ether. The method comprises distilling-said mixture in the presence of a polar or non-polar extractive agent.

Allyl ethers of trimethylolpropane are well known compounds. The mono and diallyl ethers are frequently used in for instance the preparation of unsaturated polyesters and alkyds, acrylic monomers and other high tech coating areas. The most interesting application areas for the triallyl ether include superabsorbant fibrous materials, cosmetic preparations, thickeners and reactive diluents. The development of applications wherein trimethylolpropane triallyl ether would substantially contribute to improved properties have, due to a limited availability of said product, been obstructed and involuntarily slowed down. The demand for trimethylolpropane triallyl ether is accordingly high why production and/or recovery methods increasing the availability are of substantial interest.

Trimethylolpropane triallyl ether can as corresponding diallyl ether be produced according to known allylation processes (cf. for example GB-A-1 473 024) or in accordance with the present invention recovered from for instance commercially available trimethylolpropane diallyl ether grades and secondary products from said processes. Most trimethylolpropane mono and diallyl ether grades normally comprise a certain amount, such as 1%, 5%, 10%, 15% or 20% by weight or mole of the triallyl ether as by-product. The triallyl ether is in many monoallyl and diallyl ether applications, such as polyesters, alkyds and acrylic monomers, of no or insignificant value. Recovery of said by-product, and pendant purification of the mono and/or diallyl ether is very difficult as trimethylolpropane diallyl ether and trimethylolpropane triallyl ether have similar solubility and volatility and furthermore form a fraction having an azeotropic composition comprising about 70% by weight of diallyl ether and about 30% by weight of triallyl ether.

The present invention provides quite unexpectedly a method which suitably is used at recovery of trimethylolpropane triallyl ether and/or trimethylolpropane diallyl ether from a mixture comprising said triallyl ether and said diallyl ether. It has been found that said azeotropic mixture between trimethylolpropane diallyl and triallyl ether can be broken by addition of a third substance, a so called entrainer.

The method according to the present invention comprises distilling at least once a mixture comprising trimethylolpropane triallyl end diallyl ether at a pressure of less than 12 kPa, such as less than 10 kPa or even less than 1 kPa, in a distillation column in the presence of an effective amount of at least one polar or non-polar extractive agent. The extractive agent is in preferred embodiments of the invention fed continuously during the distillation, which can be either a batch distillation or a continuous distillation. The extractive agent can be fed to the still head or stillpot of the distillation equipment or preferably to the distillation column via for instance the top section or any other section of said column. The distillation procedure of the present method is in preferred embodiments performed at a reflux ratio of 0.05-15, preferably 0.1-10. The reflux ratio is defined as the ratio between distillate and reflux. The method of the present invention can advantageously be used when recovering trimethylolpropane triallyl and/or diallyl ether from by-products obtained when processing said ethers or in purification of trimethylolpropane diallyl ether grades having a certain amount of trimethylolpropane triallyl ether as by-product as well as in purification of trimethylolpropane triallyl ether grades having a certain amount of trimethylolpropane diallyl ether as by-product. Trimethylolpropane triallyl ether and/or trimethylolpropane diallyl ether can thus individually be obtained from received distillate or received distillation residue and optionally further processed, including further extractive distillation or distillations, to yield substantially pure products.

Extractive distillation in general is disclosed and discussed in "Perry's Chemical Engineers' Handbook", 6th edition, McGraw-Hill Book Company 1984, page 13-53 through 13-57 and in "Handbook of Separation Techniques for Chemical Engineers", Phillip A. Schweitzer, McGraw-Hill Book Company, 1979, page 1-135 through 1-143.

Polar extracting agents include, in various embodiments of the present invention, polar compounds such as alcohols, alkanolamines, sulfolanes and/or mixtures thereof and therewith. The selection of extractive agent is dependent on its boiling temperature, which preferably is a high boiling compound having a boiling point close to the boiling point of trimethylolpropane triallyl and diallyl ether. Alcohols are for instance diols, triols and polyols, such as ethylene glycols, propylene glycols, 2-alkyl and 2,2-dialkyl substituted 1,3-propanediols, trimethylolalkanes, for instance trimethylolethane and trimethylolpropane, pentaerythritols and higher alcohols. Alkanolamines are for instance mono, di and triethanolamine and di and triisopropanolamine.

Suitable polar extracting agents may also be found among dimers, trimers and polymers of for instance said alcohols, mixtures comprising two or more alcohols, including said dimers, trimers and polymers, mixtures comprising two or more alkanolamines and mixtures comprising at least one alcohol or dimer, trimer or polymer thereof and at least one alkanolamine.

Suitable non-polar extractive agents are substantially to be found among linear and branched hydrocarbons having at least 10 carbon atoms in its primary chain, such as hexadecane, heptadecane and octadecane.

The necessary or most suitable amount of extraction agent is dependent on a number of properties, such as desired purity of recovered trimethylolpropane triallyl and/or diallyl ether, the molar ratio triallyl ether to diallyl ether in the mixture, the amount and type of other products and by-products in the mixture affecting the extractive distillation, the set-up of the distillation equipment, the length of the distillation column, the reflux ratio, the placing of the extractive agent feed, the pressure under which the mixture is distilled and similar properties. The effective amount of extractive agent is a matter of optimisation and can normally be determined empirically. A suitable amount is, however, often to be found within a molar ratio trimethylolpropane triallyl ether to polar extractive agent or trimethylolpropane diallyl ether to non-polar extractive agent of 1:5 to 5:1. The effective or most effective amount of extractive agent can, furthermore, differ during various stages of the distillation.

The method of the present invention can easily be developed and adapted to encompass extractive distillation of mixtures comprising at least one non-polar and at least one polar allyl ether forming mixtures similar to trimethylolpropane diallyl ether and trimethylolpropane triallyl ether, that is an azeotropic mixture or a mixture otherwise inseparable or difficult to separate by ordinary distillation. Full and partial allyl ethers can for instance, besides disclosed trimethylolpropane allyl ethers, be produced through allylation of most known alcoholic compounds, such as ethylene glycols, propylene glycols, neopentyl glycol, 2,2-alkyl- 1,3-propanediols, trimethylolethane, trimethylolbutane, pentaerythritol, anhydroenneaheptitol, sugar alcohols, hydroxyfunctional carboxylic acids, such as 2,2-dimethylolpropionic acid, and similar compounds as well as through allylation of dimers, trimers and polymers of said alcoholic compounds.

Discussed and other objects and the attendant advantages will be more fully understood from the following detailed description, taken in conjunction with below embodiment examples 1-2.

Example 1 shows evaporation of a mixture, comprising trimethylolpropane triallyl ether and diallyl ether, using trimethylolpropane, triethanolamine and hexadecane as extractive agent in comparison with evaporation without using an extractive agent and Example 2 discloses an extractive distillation of a mixture, comprising trimethylolpropane triallyl and diallyl ether, using trimethylolpropane as extractive agent.

### Example 1

A mixture comprising trimethylolpropane diallyl ether and trimethylolpropane triallyl ether at a weight percentage of 73:27 was together with an extractive agent charged in 500 ml distillation flasks connected to a cooler and a receiver. The mixture was at a temperature of 130-135°C and a pressure of 1-1.2 kPa allowed to evaporate to respective receiver. A control without extractive agent was treated in the same manner.

The following amounts were charged:

| | |
|---|---|
| Sample 1 | 300 ml of the mixture and 200 ml of molten trimethylolpropane. |
| Sample 2 | 320 ml of the mixture and 180 ml triethanolamine. |
| Sample 3 | 220 ml of the mixture and 280 ml of hexadecane. |
| Control | 500 ml of the mixture. |

The weight percentage of trimethylolpropane triallyl ether in the distillate collected in respective receiver was after approximately 60 minutes of evaporation determined to be:

| | |
|---|---|
| Sample 1 | 44% by weight |
| Sample 2 | 41% by weight |
| Sample 3 | 23% by weight |
| Control | 31% by weight |

The result clearly indicates that the azeotropic trimethylolpropane diallyl triallyl ether mixture is broken in Samples 1-3, contrary to the control sample.

### Example 2

500 ml of a mixture comprising 2.1% by weight of volatile compounds, 1.2% by weight of trimethylolpropane monoallyl ether, 66.2% by weight of trimethylolpropane diallyl ether, 24.8% by weight of trimethylolpropane triallyl ether, 0.2% by weight of trimethylolpropane and 5.5% by weight of high boiling compounds was charged in a the stillpot of a distillation equipment comprising a 2000 ml stillpot, a stirrer, a thermometer, a 200 cm distillation column with 1 m of Sulzer BX™ packing, a cooler and receivers for the distillate divided into pre-fraction, main fractions and after-fractions. Trimethylolpropane as extractive agent was during the distillation continuously fed from the top of the distillation column. Collecting of a pre-fraction was preceded by a one hour total reflux at a temperature of ≈ 125°C and a pressure of 0.6-0.8 kPa.

The following result was obtained:

| | Distillation^{*1} time hrs | Distillate flow g/min | Temperature^{*2} °C | Pressure kPa | Reflux ratio | TMP^{*3} g/min | TMPTE^{*4} weight% |
|---|---|---|---|---|---|---|---|
| Pre-fraction | 0.4 | 8 | 124-126 | 0.6-0.8 | 10 | 2 | 55 |
| Main fractions | 1.5 | 10-12 | 126-136 | 0.6-0.8 | 2 | 4-8 | 66-81 |
| After-fractions | 1.5 | 5-0.1 | 136-178 | 0.6-0.8 | 4 | 2-8 | 40-0 |

| | | | | | | | |
|---|---|---|---|---|---|---|---|
| *1: Respective fraction or fractions. | | | | | | | |
| *2: Measured in the stillpot. | | | | | | | |
| *3: Trimethylolpropane (TMP) fed to the column top. | | | | | | | |
| *4: Weight percentage trimethylolpropane triallyl ether (TMPTE) on total weight of trimethylolpropane triallyl ether and trimethylolpropane diallyl ether in the distillate fractions. | | | | | | | |

As can be seen from above result, the extractive distillation gave good to excellent separation of trimethylolpropane triallyl and diallyl ether.

## Claims

1. A method in recovery of trimethylolpropane triallyl ether and/or trimethylolpropane diallyl ether from a mixture comprising said triallyl ether and said diallyl ether **characterised in, that** said method comprises distilling said mixture at a pressure of less than 12 kPa in a distillation column in the presence of an effective amount of at least one polar or non-polar extractive agent.

2. A method according to Claim 1 **characterised in, that** said mixture is distilled at a pressure of less than 10 kPa, such as less than 1 kPa.

3. A method according to Claim 1 or **characterised in**, a reflux ratio of 0.05-15, preferably 0.1-10.

4. A method according to any of the Claims 1-3 **characterised in, that** said mixture is distilled in a batch distillation.

5. A method according to any of the Claims 1-3 **characterised in, that** said mixture is distilled in a continuous distillation.

6. A method according to any of the Claims 1-5 **characterised in, that** said extractive agent is fed continuously.

7. A method according to Claim 6 **characterised in, that** said extractive agent is fed to the distillation column, preferably to a top section thereof.

8. A method according to any of the Claims 1-7 **characterised in, that** said extractive agent is a polar substance comprising at least one alcohol and/or at least one alkanolamine.

9. A method according to Claim 8 **characterised in, that** the extractive agent is trimethylolpropane, triethanolamine or a mixture thereof.

10. A method according to Claim 8 or 9 **characterised in**, a molar ratio trimethylolpropane triallyl ether to extractive agent of 1:5 to 5:1.

11. A method according to any of the Claims 1-7 **characterised in, that** said extractive agent is a non-polar substance comprising at least one linear or branched aliphatic hydrocarbon having at least 10 carbon atoms, such as hexadecane, heptadecanc and/or octadecane.

12. A method according to Claim 11 **characterised in**, a molar ratio trimethylolpropane diallyl ether to extractive agent of 1:5 to 5:1.

13. A method according to any of the Claims 1-12 **characterised in, that** a trimethylolpropane diallyl ether grade is purified from trimethylolpropane triallyl ether by-product.

14. A method according to any of the Claims 1-13 **characterised in, that** a trimethylolpropane triallyl ether grade is purified from trimethylolpropane diallyl ether by-product.

15. A method according to any of the Claims 1-14 **characterised in, that** trimethylolpropane triallyl ether and/or trimethylolpropane diallyl ether individually are obtained or yielded from received distillate or received distillation residue and optionally further purified.

## Patentansprüche

1. Verfahren zur Wiedergewinnung von Trimethylolpropantriallylether und/oder Trimethylolpropandiallylether aus einer Mischung umfassend den Triallylether und den Diallylether, **dadurch gekennzeichnet, daß** das Verfahren Destillieren der Mischung bei einem Druck von weniger als 12 kPa in einer Destillationssäule in Gegenwart einer wirksamen Menge von zumindest einem polaren oder nicht-polaren Extraktivmittel umfaßt.

2. Verfahren gemäß Anspruch 1, **dadurch gekennzeichnet, daß** die Mischung bei einem Druck von weniger als 10 kPa, wie weniger als 1 kPa, destilliert wird.

3. Verfahren gemäß Anspruch 1 oder 2 **gekennzeichnet durch** ein Rückflußverhältnis von 0,05 bis 15, bevorzugt 0,1 bis 10.

4. Verfahren gemäß einem der Ansprüche 1 bis 3, **dadurch gekennzeichnet, daß** die Mischung in einer Batch-Destillation destilliert wird.

5. Verfahren gemäß einem der Ansprüche 1 bis 3, **dadurch gekennzeichnet, daß** die Mischung in einer kontinuierlichen Destillation destilliert wird.

6. Verfahren gemäß einem der Ansprüche 1 bis 5, **dadurch gekennzeichnet, daß** das Extraktivmittel kontinuierlich zugeführt wird.

7. Verfahren gemäß Anspruch 6, **dadurch gekennzeichnet, daß** das Extraktivmittel der Destillationssäule, bevorzugt im oberen Bereich davon, zugeführt wird.

8. Verfahren gemäß einem der Ansprüche 1 bis 7, **dadurch gekennzeichnet, daß** das Extraktivmittel eine polare Substanz, umfassend zumindest einen Alkohol und/oder zumindest ein Alkanolamin ist.

9. Verfahren gemäß Anspruch 8, **dadurch gekennzeichnet, daß** das Extraktivmittel Trimethylolpropan, Triethanolamin oder eine Mischung davon ist.

10. Verfahren gemäß Anspruch 8 oder 9, **gekennzeichnet durch** ein Molverhältnis Trimethylolpropantriallylether zum Extraktivmittel von 1:5 bis 5:1.

11. Verfahren gemäß einem der Ansprüche 1 bis 7, **dadurch gekennzeichnet, daß** das Extraktivmittel eine nichtpolare Substanz umfassend zumindest einen linearen oder verzweigten aliphatischen Kohlenwasserstoff mit zumindest 10 Kohlenstoffatomen, wie Hexadecan, Heptadecan und/oder Octadecan, ist.

12. Verfahren gemäß Anspruch 11, **gekennzeichnet durch** ein Molverhältnis Trimethylolpropandiallylether zum Extraktivmittel von 1:5 bis 5:1.

13. Verfahren gemäß einem der Ansprüche 1 bis 12, **dadurch gekennzeichnet, daß** eine Trimethylolpropandiallylether-Qualität von Trimethylolpropantriallylether-Nebenprodukt gereinigt wird.

14. Verfahren gemäß einem der Ansprüche 1 bis 13, **dadurch gekennzeichnet, daß** eine Trimethylolpropantriallylether-Qualität von Trimethylolpropandiallylether-Nebenprodukt gereinigt wird.

15. Verfahren gemäß einem der Ansprüche 1 bis 14, **dadurch gekennzeichnet, daß** Trimethylolpropantriallylether und/oder Trimethylolpropandiallylether einzeln aus dem erhaltenen Destillat oder erhaltenen Destillationsrückstand erhalten oder gewonnen und optional weitergereinigt werden.

## Revendications

1. Procédé de récupération d'un triallyléther de triméthylolpropane et/ou d'un diallyléther de triméthylolpropane, provenant d'un mélange comprenant ledit triallyléther et ledit diallyléther, **caractérisé en ce qu'**il comprend la distillation dudit mélange sous une pression inférieure à 12 kPa dans une colonne de distillation en présence d'une quantité efficace d'au moins un agent d'extraction polaire ou non polaire.

2. Procédé selon la revendication 1, **caractérisé en ce que** l'on distille ledit mélange sous une pression inférieure à 10 kPa, telle que inférieure à 1 kPa.

3. Procédé selon la revendication 1 ou 2, **caractérisé par** un taux de reflux de 0,05 à 15, de préférence de 0,1 à 10.

4. Procédé selon l'une quelconque des revendications 1 à 3, **caractérisé en ce que** l'on distille ledit mélange en discontinu.

5. Procédé selon l'une quelconque des revendications 1 à 3, **caractérisé en ce que** l'on distille ledit mélange en continu.

6. Procédé selon l'une quelconque des revendications 1 à 5, **caractérisé en ce que** l'on introduit ledit agent d'extraction en continu.

7. Procédé selon la revendication 6, **caractérisé en ce que** l'on introduit ledit agent d'extraction dans la colonne de distillation, de préférence au niveau de sa section supérieure.

8. Procédé selon l'une quelconque des revendications 1 à 7, **caractérisé en ce que** ledit agent d'extraction est une substance polaire comprenant au moins un alcool et/ou au moins une alcanolamine.

9. Procédé selon la revendication 8, **caractérisé en ce que** ledit agent d'extraction est le triméthylolpropane, la triéthanolamine ou un mélange de ces derniers.

10. Procédé selon la revendication 8 ou 9, **caractérisé par** un rapport molaire du triallyléther de triméthylolpropane à l'agent d'extraction allant de 1:5 à 5:1.

11. Procédé selon l'une quelconque des revendications 1 à 7, **caractérisé en ce que** ledit agent d'extraction est une substance non polaire comprenant au moins un hydrocarbure aliphatique, linéaire ou ramifié, comportant au moins 10 atomes de carbone, tel que l'hexadécane, l'heptadécane et/ou l'octadécane.

12. Procédé selon la revendication 11, **caractérisé par** un rapport molaire du diallyléther de triméthylolpropane à l'agent d'extraction allant de 1:5 à 5:1.

13. Procédé selon l'une quelconque des revendications 1 à 12, **caractérisé en ce que** l'on purifie une qualité de diallyléther de triméthylolpropane à partir d'un produit secondaire triallyléther de triméthylolpropane.

14. Procédé selon l'une quelconque des revendications 1 à 13, **caractérisé en ce que** l'on purifie une qualité de triallyléther de triméthylolpropane à partir d'un produit secondaire diallyléther de triméthylolpropane.

15. Procédé selon l'une quelconque des revendications 1 à 14, **caractérisé en ce que** l'on obtient individuellement le diallyléther de triméthylolpropane et/ou le triallyléther de triméthylolpropane à partir d'un distillât récupéré ou d'un résidu de distillation récupéré, et éventuellement on le purifie encore.
